# EUROPÄISCHE PATENTANMELDUNG

(11) **EP 0 751 105 A2**
(43) Veröffentlichungstag der Anmeldung: **02.01.1997**
(21) Anmeldenummer: 96109679.9
(22) Anmeldetag: 17.06.1996
(51) Int. Cl.: C07B 33/00, C07D 471/04, C07C 229/58

(54) **Verfahren zur Oxidation organischer Verbindungen**

(30) Priorität: 28.06.1995 DE 19523445
(71) Anmelder: BAYER AG, 51368 Leverkusen (DE)
(72) Erfinder: Schneider, Jürgen, Dr., 51519 Odenthal (DE); Schütze, Detlef-Ingo, Dr., 51061 Köln (DE); Krügermann, Claus, 51381 Leverkusen (DE)

(57) **Zusammenfassung**

Organische Verbindungen, insbesondere Farbstoffe und Vorprodukte werden mit Sauerstoff oder sauerstoffhaltigen Gasen oxidiert, wobei über die Sauerstoffkonzentration im Abgas die Oxidationsreaktion gesteuert wird.

## Beschreibung

Die Erfindung betrifft ein verbessertes Verfahren zur Oxidation organischer Verbindungen mit Sauerstoff oder sauerstoffhaltigen Gasen, wobei die Reaktion über die Sauerstoffkonzentration im Abgas gesteuert wird.

In der Literatur ist eine große Anzahl von Verfahren zur Oxidation und ihrer Kontrolle beschrieben.

Nach Liebermann (Liebigs Annalen, Band 404 (1914), S. 272) werden z.B. die 2,5-Diarylaminoterephthalsäuren durch Oxidation von 2,5-Diarylamino-3,6-dihydroterephthalsäuredialkylestern mit Jod in alkoholischer Lösung oder mit Luftsauerstoff in Eisessig/Alkohol und durch Verseifen der so erhaltenen und isolierten 2,5-Diarylaminoterephthalsäuredialkylester mit alkoholisch-wäßriger Lauge erhalten.

Um die bekannte Verwendung von aromatischen Nitroverbindungen vermeiden zu können, wurden weitere Versuche unternommen, Verfahren für die Oxidation mit Sauerstoff bzw. sauerstoffhaltigen Gasen zu entwickeln.

So werden Verfahren für die Oxidation durch Sauerstoff oder sauerstoffhaltige Gase für 6,13-Dihydrochinacridone beschrieben, bei denen die Reaktion in Alkohol/Wasser/Natriumhydroxid unter Zusatz von geringen Mengen Chinonen, wie beispielsweise Naphthochinonen, Phenanthrenchinon, Anthrachinon oder deren Sulfon- oder Carbonsäuren (DE-AS 1 210 110) oder mit größeren Anteilen an Eisen-, Nickel- oder Kobaltsalzen durchgeführt wird (US-A 3 738 988). In der DE-AS 1 114 285 wird die Oxidation der 2,5-Diarylamino-3,6-dihydroterephthalsäuredialkylester in waßrig-alkoholischen Laugen unter Zusatz von geringen Mengen Chinonsulfonsäuren, wie Naphthochinon-, Anthrachinon- und Phenanthrenchinonsulfonsäuren als Sauerstoffüberträger mit Luft vorgenommen.

Die für die Oxidation von 2,5-Diarylamino-3,6-dihydroterephthalsäuredialkylestern in EP-A-0 363 756 und der 6,13-Dihydrochinacridone in EP-A-363 759 beschriebene Verwendung von quartären Ammoniumsalzen als Sauerstoffüberträger ermöglicht zwar die Herstellung von 2,5-Diarylaminoterephthalsäuren bzw. substituierten Chinacridonen, erfordert aber einen erheblichen ökologischen Aufwand. Das in WO 92/09 558 beschriebene Oxidationsverfahren für 2,5-Diarylamino-3,6-dihydroterephthalsäuredialkylester mit Sauerstoff ist ebenfalls sehr aufwendig.

Die Erfindung betrifft ein Verfahren zur Oxidation organischer Verbindungen mit Sauerstoff oder sauerstoffhaltigen Gasen, vorzugsweise Luft, in Lösung oder Suspension in einem Reaktor, dadurch gekennzeichnet, daß die Sauerstoffkonzentration im Abgas des Reaktors, vorzugsweise on line, gemessen wird und über die Veränderung der Sauerstoffkonzentration die Oxidation gesteuert wird.

Bevorzugt wird die organische Verbindung zu einem Farbstoff, einem Pigment oder zu Vorstufen davon oxidiert.

Die Steuerung erfolgt vorzugsweise so, daß der Sauerstoffgehalt im Abgas gemessen wird, wobei eine Zunahme der Sauerstoffkonzentration im Abgas, die auch das Ende der Oxidationsreaktion anzeigt, über geeignete Sonden eine weitere Zufuhr von Sauerstoff unterbricht.

Bevorzugte Reaktoren sind Rührwerkkessel, druckfest bis 10 bar Überdruck, aus unterschiedlichem Material wie stahl-emailliert und Edelstahl.

Die Messung der Sauerstoffkonzentration erfolgt vorzugsweise so, daß ein Teilstrom der Abluft zur Sauerstoff-Sonde geführt wird.

Die Konzentration des Sauerstoffs im Abgas kann über die Messung der paramagnetischen Eigenschaften des Sauerstoffs, durch Ionisation des Sauerstoffs und Messung des Ionenstroms an einem Platin/ZrO₂-Elektrodensystem und vorzugsweise mit einer elektrochemischen Meßzelle bestimmt werden. Besonders bevorzugte elektrochemische Meßzellen sind charakterisiert durch eine weitgehend von der Temperatur unbeeinflußte, lineare Charakteristik zwischen Sauerstoff-Gehalt und Meßstrom. Ein Langzeitbetrieb hat keinen Einfluß auf die Meßgenauigkeit, führt lediglich zu einer Einengung des Meßbereichs.

Bevorzugte Reaktionsmedien sind alkoholisch-alkalische oder alkoholisch-wäßrigalkalische Lösungen oder Suspensionen, gegebenenfalls in Gegenwart eines sauerstoffübertragenden Mittels.

Besonders bevorzugte Lösungen und Suspensionen enthalten als Lösungsmittel anorganische Mineralsäuren wie Schwefelsäure; Alkohole wie Methanol, Ethanol, Propanol, Isopropanol, Butanol, Glykol, Methylglykol; organische Lösungsmittel wie alkylierte Benzole, Amine wie Anilin, alkylierte Arylamino-Verbindungen.

Bevorzugte sauerstoffübertragende Mittel sind Chinone wie Anthrachinon, Phenanthrenchinon, Naphthochinon und Chloranil sowie deren Sulfon- und Carbonsäuren, die auch als Salze Verwendung finden können. Besonders bevorzugt ist die Verwendung von Anthrachinonmono- und -disulfonsäuren, bzw. deren Salzen; ganz besonders bevorzugt wird Anthrachinon-2-sulfonsäure(salz) eingesetzt.

Die Reaktionen können bei beliebigen Drucken und Temperaturen durchgeführt werden, bevorzugt sind Temperaturen von 0 bis 150°C und 1 bis 11 bar.

Desweiteren ist eine zusätzliche Zuführung eines Inertgases, z.B. Stickstoff, zur Absenkung der Sauerstoffkonzentration unter die explosible Grenze von 8 % möglich aber nicht erforderlich, da bei den angegebenen Reaktionsbedingungen der Sauerstoff spontan und vollkommen verbraucht wird.

Die Oxidationen werden vorzugsweise bei 70 bis 130°C, besonders bevorzugt beim Siedepunkt des Reaktionsgemischs unter Druck, durchgeführt. Gegebenenfalls wird drucklos gearbeitet.

Insbesondere dient das Verfahren zur Herstellung von
1. Chinacridonen der Formel 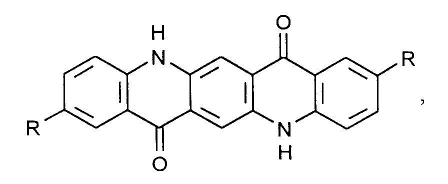 in der
   - R =: H, CH₃, OCH₃, Cl ist.
2. 2,5-Diarylaminoterephthalsäuren der Formel 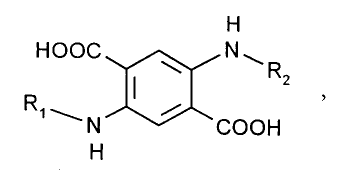 in der 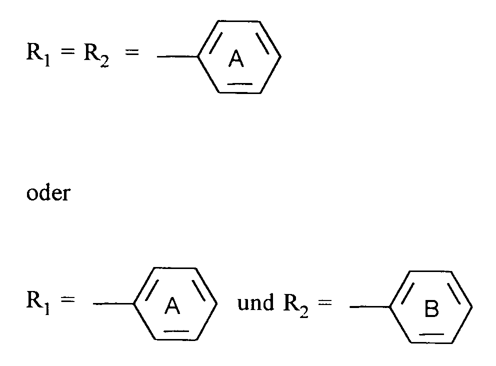 sind,
   wobei die Ringe A und B durch 1 bis 4 Substituenten aus der Reihe C₁-C₄-Alkyl, Chlor, Fluor, C₁-C₄-Alkoxy, gegebenenfalls durch C₁-C₄-Alkyl mono- oder disubstituiertes Carbamoyl, Trifluormethyl, Carboxy, Nitro substituiert sein können oder an sie ein aromatischer oder heteroaromatischer Ring ankondensiert sein kann, wobei man vorzugsweise 2,5-Diarylamino-3,6-dihydroterephthalsäuredialkylester der Formel 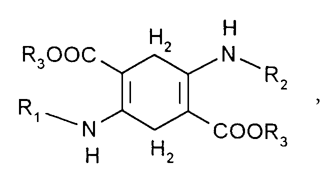 in der
   R₁, R₂ die oben angegebenen Bedeutungen haben und
   R₃ für Alkyl, vorzugsweise C₁-C₈-Alkyl, z.B. Methyl, Ethyl, i-Propyl, steht oder entsprechende Bildungsgemische in die Oxidation einsetzt. Gleichzeitig mit der Oxidation werden die Dialkylester verseift und gehen als Di-Natriumsalz in Lösung.
3. Anthrachinonen, insbesondere Hydroxyanthrachinonen der Formel 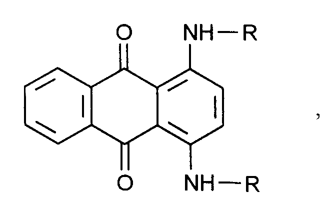 wobei R = unabhängig voneinander ein aliphatischer, alicyclischer oder Arylamino-Rest ist und das Anthrachinon durch eine oder mehrere OH-Gruppen substituiert sein kann
   aus entsprechenden Dihydroanthrachinonen.
4. Dioxazinen der Formel 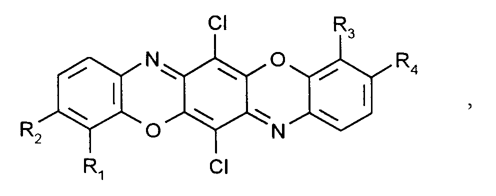 wobei bedeuten
   - R₁, R₃:: unabhängig voneinander -SO₃H, -SO₂-CH₂-CH₂-SO₃H,
   - R₂, R₄:: unabhängig voneinander -NH₂, -NH-CH₂-CH₂-SO₃H
   durch oxidativen Ringschluß.

Ganz besonders bevorzugt ist die Oxidation von organischen Verbindungen, insbesondere 6,13-Dihydrochinacridonen bzw. 2,5-Diarylamino-3,6-dihydroterephthalsäuredialkylestern mit Sauerstoff oder sauerstoffhaltigen Gasen.

Das erfindungsgemäße Verfahren läßt sich z.B. wie folgt ausführen:
Zu dem Bildungsgemisch zur Herstellung der 2,5-Diarylamino-3,6-dihydroterephthalsäureester, das aus Succinylobernsteinsäuredialkylester, Arylamin, Alkohol und beispielsweise etwas Eisessig zur Kondensation besteht, wird ein Alkalihydroxid oder eine Lösung eines Alkalihydroxids in Wasser, einem Sauerstoffüberträger, beispielsweise Anthrachinonsulfonsäure, hinzufügt, das Gemisch erhitzt und durch Durchleiten von Sauerstoff oder sauerstoffhaltigen Gasen, wie beispielsweise Luft, unter Druck oxidiert.

Während der Oxidation läßt sich der Sauerstoff-Gehalt im Reaktionsgefäß durch eine Kurve darstellen, deren Verlauf den sofortigen Verbrauch des zur Reaktion angebotenen Sauerstoffs zeigt. In keiner Phase der Reaktion kann sich ein explosibles Gemisch mit ^{C}O₂>8 Vol.-% bilden, da das Reaktionsende durch den steilen Anstieg des Rest-Sauerstoff-Gehaltes im Abgas eindeutig angezeigt wird.

Die genaue Kontrolle der Sauerstoff-Dosierung, um den Anstieg des O₂-Gehaltes im Gasraum über 8 Vol.-% und somit in den explosiblen Bereich zu vermeiden, übernimmt eine Sauerstoff-Meßsonde.

Als Arylamine werden bevorzugt Anilin, p-Toluidin, p-Chloranilin, p-Anisidin oder p-Phenetidin eingesetzt. Als Alkalihydroxide sind Natriumhydroxid und Kaliumhydroxid besonders bevorzugt.

Als Alkohole kommen vorzugsweise Methanol, Ethanol, i-Propanol, n-Butanol, Glykole oder Glykolether, z.B. Ethylenglykol, Ethylenglykolmonomethylether und Polyglykole sowie Gemische dieser Alkohole in Betracht.

Als sauerstoffübertragende Mittel werden vorzugsweise eingesetzt: Chinone wie Anthrachinon, Phenanthrenchinon, Naphthochinon und Chloranil sowie deren Sulfon- und Carbonsäuren, die auch als Salze Verwendung finden.

### Beispiel 1

### Di-p-toluidino-terephthalsäure

362 Teile Methanol, 50 bis 100 Teile Dimethylsuccinylsuccinat = DMSS, 55 bis 110 Teile p-Toluidin und 0,5 bis 0,8 Teile Schwefelsäure 96 %ig wurden in einem Druckreaktor 5 Std. auf 95°C erhitzt. Es wurde abgekühlt und eine Lösung von 45 bis 80 Teilen Ätzkali in 55 bis 95 Teilen Wasser und 3,9 bis 6,5 Teile Anthrachinon-2-sulfonsäure zugegeben. Anschließend wurde im druckfest verschlossenen Reaktor auf 95°C erhitzt. Dabei stieg der Druck auf ca. 1,2 bar an. Dann wurden 6,8 bis 10 l Luft pro Stunde in die Schmelze eingeleitet, wobei der Druck durch ein Druckhalteventil 1 bar über dem Ausgangsdruck gehalten wurde. Temperatur, Gasmenge und Druck wurden so lange gehalten, bis die angeschlossene Sauerstoff-Meßsonde einen deutlichen Anstieg der Sauerstoffkonzentration im Abgas anzeigte. Dauer: ca. 4 bis 5 Std.

Das Reaktionsgemisch wurde mit 420 Teilen Wasser verdünnt, mit 3 Teilen Celite geklärt, das Filtrat mit 30 Teilen Schwefelsäure 48 %ig versetzt, die Fällung isoliert und neutralgewaschen.
Ausbeute: 81,9 bis 136 Teile (89 bis 91 % der Theorie).

### Beispiel 2

### Di-anilino-terephthalsäure

365 Teile Methanol, 112 Teile DMSS, 110 Teile Anilin und 41 Teile Eisessig wurden in einem Druckreaktor 2 Std. auf 104 bis 105°C geheizt. Nach Abkühlen wurden eine Lösung aus 113,5 Teilen Ätznatron in 233,5 Teilen Wasser und 2,4 Teile Anthrachinon-2-sulfonsäure zugegeben.

Anschließend wurde im druckfest verschlossenen Reaktor auf 75°C erwärmt. Gleichzeitig wurden 5 l Luft pro Stunde in die Schmelze und 5 l Stickstoff in den Gasraum geleitet, bis die angeschlossene Sauerstoff-Meßsonde einen deutlichen Anstieg der Sauerstoffkonzentration im Abgas anzeigt. Der Druck von 1 bar wurde durch ein Druckhalteventil gehalten. Reaktionsdauer: etwa 6 Std. Es wurde abgekühlt, mit 840 Teilen Wasser verdünnt, mit 6,9 Teilen eines Klärungsmittels geklärt und das Filtrat mit 148 Teilen Schwefelsäure 96 %ig versetzt. Die Fällung wurde isoliert, mit Wasser neutralgewaschen und getrocknet.
Ausbeute: 161 Teile = 97 % der Theorie.

### Beispiel 3

### Di-p-chloranilino-terephthalsäure

362,4 Teile Methanol, 81,9 Teile DMSS, 101,9 Teile p-Chloranilin und 31,3 Teile Eisessig wurden in einem Druckreaktor 4 Std. auf 95°C erhitzt. Nach Abkühlen wurden 151 Teile Methanol, 207,6 Teile 45 %ige Kalilauge und 1,1 Teile Anthrachinon-2-sulfonsäure zugegeben. Anschließend wurde im druckfest verschlossenen Reaktor auf 95°C erhitzt. Dabei stellt sich ein Druck von ca. 1 bar ein. Gleichzeitig wurden 17 l Luft pro Stunde bis zu einem Druck von 1 bar über dem Ausgangsdruck eingeleitet. Temperatur, Luftmenge und Druck wurden 6 Std. gehalten, bis die angeschlossene Sauerstoff-Meßsonde einen deutlichen Anstieg der Sauerstoffkonzentration im Abgas anzeigt. Das Reaktionsgemisch wurde abgekühlt, mit 755 Teilen Wasser verdünnt, mit 5,7 Teilen Celite geklärt, das Filtrat mit 160,7 Teilen konz. Salzsäure kongosauer eingestellt. Die gefällte Di-p-chloranilino-terephthalsäure wurde abgesaugt, mit Wasser neutralgewaschen und getrocknet.
Ausbeute: 140,1 Teile eff. = 93,6 % der Theorie eff.

### Beispiel 4

### Di-p-anisidino-terephthalsäure

362,4 Teile Methanol, 46,6 Teile DMSS, 58,7 Teile p-Anisidin und 18,1 Teile Eisessig wurden in einem Druckreaktor 5 Std. auf 97 bis 100°C erhitzt. Es wurde abgekühlt, und 94,9 Teile 50 %ige Natronlauge, 51 Teile Wasser und 1 Teil Anthrachinon-2-sulfonsäure zugegeben. Anschließend wurde im druckfest verschlossenen Reaktor auf 75°C erwärmt und gleichzeitig 5 bis 6 Stunden lang 6,5 l Luft pro Stunde bis zu einem Überdruck von 2,2 bar eingeleitet, bis die angeschlossene Sauerstoff-Meßsonde einen deutlichen Anstieg der Sauerstoffkonzentration im Abgas anzeigte. Es wurde abgekühlt und das Reaktionsgemisch mit 345 Teilen Wasser verdünnt, mit 2,6 Teilen eines Klarungsmittels bei 70°C geklärt, das Filtrat bei gleicher Temperatur mit 130,9 Teilen konz. Salzsäure kongosauer gestellt. Die ausgefallene Di-p-anisidino-terephthalsäure wurde warm abgesaugt, mit Wasser neutralgewaschen und getrocknet.
Ausbeute: 74,8 Teile eff. = 93,7 % der Theorie eff.

### Beispiel 5

### γ-Chinacridon

24 Teile Ätznatron wurden in 228,8 Teilen Methanol gelöst, 60 Teile Dihydrochinacridon eingetragen und mit 96 Teilen Methanol verdünnt. Der Ansatz wurde auf 60°C geheizt und 1 Std. verrührt. Anschließend wurde eine Lösung aus 6 Teilen Dispergiermittel SS in 442 Teilen Wasser zum Ansatz gegeben, 1,5 Teile Anthrachinon-2-sulfonsäure zugegeben. Der Reaktor wurde druckfest verschlossen, auf 75°C geheizt, dann wurde 8 Stunden 6 l Luft pro Stunde bis 1 bar Überdruck eingeleitet, bis die angeschlossene Sauerstoff-Meßsonde einen deutlichen Anstieg der Sauerstoffkonzentration im Abgas anzeigte. Der Druck wurde über ein Druckhalteventil konstant gehalten. Der Ansatz wurde bei 60°C isoliert und mit Wasser neutralwaschen.

Das feuchte Produkt wurde in 300 Teile Wasser eingetragen, mit Wasser auf 630 Teile aufgefüllt, mit 2 Teilen Schwefelsäure 50 % angesäuert, 1 Std. auf 80°C geheizt, isoliert, mit Wasser neutralgewaschen und getrocknet.
Ausbeute: 57 Teile = 95 bis 96 % der Theorie.

## Patentansprüche

1. Verfahren zur Oxidation organischer Verbindungen mit Sauerstoff oder sauerstoffhaltigen Gasen in Lösung oder Suspension in einem Reaktor, dadurch gekennzeichnet, daß die Sauerstoffkonzentration im Abgas des Reaktors gemessen wird und über die Veränderung der Sauerstoffkonzentration die Oxidation gesteuert wird.

2. Verfahren nach Anspruch 1, dadurch gekennzeichnet, daß die organische Verbindung zu einem Farbstoff, Pigment oder einer Vorstufe dazu oxidiert wird.

3. Verfahren nach wenigstens einem der vorhergehenden Ansprüche, dadurch gekennzeichnet, daß ein Dihydrochinacridon zu einem Chinacridon der folgenden Formel oxidiert wird 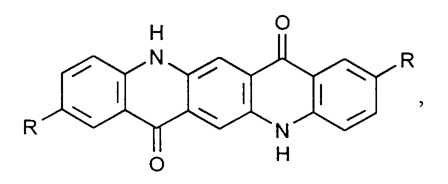 in der
R = H, CH₃, OCH₃, Cl ist.

4. Verfahren nach Anspruch 1, dadurch gekennzeichnet, daß ein Dihydroterephthalsäureester zu einer 2,5-Diarylaminoterephthalsäure der folgenden Formel oxidiert wird 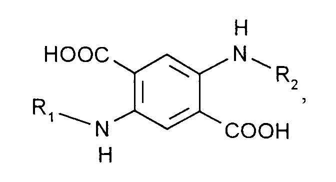 in der 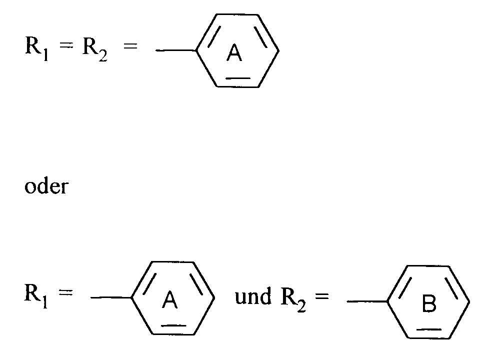 sind,
wobei die Ringe A und B durch 1 bis 4 Substituenten aus der Reihe C₁-C₄-Alkyl, Chlor, Fluor, C₁-C₄-Alkoxy, gegebenenfalls durch C₁-C₄-Alkyl mono- oder disubstituiertes Carbamoyl, Trifluormethyl, Carboxy, Nitro substituiert sein können oder an sie ein aromatischer oder heteroaromatischer Ring ankondensiert sein kann.

5. Verfahren nach wenigstens einem der vorhergehenden Ansprüche, dadurch gekennzeichnet, daß die Konzentration des Sauerstoffs mit einer elektrochemischen Meßzelle gemessen wird.

6. Verfahren nach wenigstens einem der vorhergehenden Ansprüche, dadurch gekennzeichnet, daß als sauerstoffhaltiges Gas Luft verwendet wird und die Sauerstoffkonzentration on-line gemessen wird.
